Europäisches Patentamt

European Patent Office

Office européen des brevets

(19)

(11) Veröffentlichungsnummer: **0 153 656**
A1

(12) # EUROPÄISCHE PATENTANMELDUNG

(21) Anmeldenummer: 85101519.8

(22) Anmeldetag: 13.02.85

(51) Int. Cl.⁴: **C 07 D 263/58**

(30) Priorität: 25.02.84 DE 3406909

(43) Veröffentlichungstag der Anmeldung:
04.09.85 Patentblatt 85/36

(84) Benannte Vertragsstaaten:
CH DE FR GB LI

(71) Anmelder: HOECHST AKTIENGESELLSCHAFT
Postfach 80 03 20
D-6230 Frankfurt am Main 80(DE)

(72) Erfinder: Arndt, Otto, Dr.
Frankfurter Strasse 38
D-6238 Hofheim am Taunus(DE)

(72) Erfinder: Papenfuhs, Theodor, Dr.
Heinrich-Bleicher-Strasse 40
D-6000 Frankfurt am Main 50(DE)

(54) Verfahren zur Herstellung von 2,6-Dichlorbenzoxazol.

(57) Verfahren zur Herstellung von 2,6-Dichlorbenzoxazol, dadurch gekennzeichnet, daß man 6-Chlorbenzoxazolon in Gemischen aus Chlor, Phosphortrichlorid und Phosphoroxidchlorid bzw. in Gemischen aus Phosphorpentachlorid und Phosphoroxidchlorid bei Temperaturen von 150 bis 170°C unter Druck umsetzt, wobei das Gewichtsverhältnis von Chlor und Phosphortrichlorid zu Phosphoroxidchlorid bzw. von Phosphorpentachlorid zu Phosphoroxidchlorid 0,15:1 bis 0,60:1 beträgt.

EP 0 153 656 A1

## Verfahren zur Herstellung von 2,6-Dichlorbenzoxazol

Die vorliegende Erfindung betrifft ein neues, vorteilhaftes Verfahren zur Herstellung von 2,6-Dichlorbenzoxazol. Die Herstellung von 2-Chlorbenzoxazol aus 2-Mercaptobenzoxazol (Benzoxazolthion) und Chlor oder chlorabspaltenden Mitteln (beispielsweise Phosphorpentachlorid) ist bekannt (vergleiche hierzu J. pr. Chemie 42, 454 (1890), Am. Chem. J. 21, 117 (1899), J. org. Chem. 23, 1500 (1958), Beilstein 27, Syst. Nr. 4195, DE-AS 1 210 617, NE-OS 6 505 511, GE 663 153; GE 1 164 413; EP 0 043 573, DE 3 207 153, DE-OS 1 670 453).

Es finden sich in der Literatur jedoch auch Angaben über den Umsatz von Benzoxazolonen (Z. Chemie 5 (1965), Nr. 5, S. 178 - 179) mit dem Chlorierungsmittel Brenzcatechylphosphortrichlorid (Z. Chemie 22 (1982), Nr. 4 S. 126 - 134), wobei man z.B. aus N-Methyl-benzoxazolon 2-Chlorbenzoxazol in einer Ausbeute von 75 % der Theorie erhält. Bei dieser Reaktion spaltet sich Methylchlorid ab. Benzoxazolon selbst ist offenbar als Ausgangsverbindung ungünstig, da die Ausbeute an 2-Chlorbenzoxazol stark erniedrigt wird durch Bildung von N-Benzoxazolylbenzoxazolon.

Gemäß Chem. Ber. 92, 1928 (1959) erhält man 2-Chlor-oxazole aus Oxazolonen-(2) mit Phosphoroxidchlorid unter Zusatz von Triethylamin. Eigene Versuche, auf diese Weise 2,6-Dichlorbenzoxazol aus 6-Chlorbenzoxazolon herzustellen, führten nicht zum Ziel.

In der Literaturstelle Heterocyclic Compounds Vol. 5, R.C. Elderfield, Verlag John Wiley (New York) und Chapman and Hall (London) p. 447 wird die Möglichkeit der Herstellung von 2-Chlorbenzoxazol aus Benzoxazolon und Phosphorpentachlorid ausgeschlossen, da sich eine Kernchlorierung nicht vermeiden läßt.

- 2 -

0153656

Gemäß EP 0 043 573 und DE-OS 3 207 153 läßt sich zwar 2,6-Dichlorbenzoxazol aus 6-Chlor-2-mercapto-benzoxazol mit bzw. ohne Lösemittel in hoher Reinheit (Fp. 49 - 50°C) und Ausbeute (91 % der Theorie) herstellen, jedoch ist die Herstellung des Ausgangsprodukts 6-Chlor-2-mercapto-benzoxazol aus 5-Chlor-2-aminophenol mit Alkalixanthogenat (DE-OS 3 008 225, DP 66 248) aufwendig. Zunächst stellt die Beseitigung oder anderweitige Verwendung des dabei anfallenden Schwefeldichlorids ein schwer lösbares Problem dar. Hinzu kommt, daß der der Patentliteratur zu entnehmende Produktionsweg über die drei folgenden problematischen Stufen verläuft:

1. Partieller Chloraustausch im 2,4-Dichlornitrobenzol zu 5-Chlor-2-nitrophenol (DE-OS 2 939 056, Beispiel 1) in Gegenwart von Phasentransferkatalysatoren auf Onium-Basis. Das Problem liegt in der Beseitigung der Zersetzungsprodukte der bei den hohen Reaktionstemperaturen thermisch nicht stabilen Katalysatoren und darin, daß eine Recycling-Technik für das 2,4-Dichlornitrobenzol eingeführt werden muß.

2. Katalytische Reduktion des 5-Chlor-2-nitrophenols zum 5-Chlor-2-aminophenol. Hierbei treten Probleme wegen Dehydrohalogenierung auf.

3. Ringschluß zum Mercapto-benzoxazol (EP 0 066 248). Die Beseitigung des Schwefelwasserstoffs ist aufwendig; außerdem entsteht Ethanol.

Demgegenüber wurde nun überraschenderweise gefunden, daß man 2,6-Dichlorbenzoxazol in vorteilhafter Weise herstellen kann, indem man 6-Chlorbenzoxazolon in Gemischen aus Chlor, Phosphortrichlorid und Phosphoroxidchlorid bzw. in Gemischen aus Phosphorpentachlorid und Phosphoroxidchlorid bei Temperaturen von 150 bis 170°C, vorzugsweise 155 - 165°C, unter Druck umsetzt, wobei das Gewichtsmengenverhältnis von Chlor und Phosphortrichlorid zu Phosphoroxidchlorid bzw. von Phosphorpentachlorid zu Phosphoroxidchlorid 0,15 : 1 bis 0,60 : 1 beträgt.

Das eingesetzte Phosphoroxidchlorid dient hierbei als Lösemittel. Es wird in dem vorstehend genannten Mengenverhältnis (zu den Chlorierungsmitteln) angewandt, um sicherzustellen, daß das eingesetzte Chlorierungsmittel (Phosphorpentachlorid bzw. Gemisch aus Chlor und Phosphortrichlorid) und der bei der Reaktion gebildete Chlorwasserstoff ausreichend verdünnt sind.

Die Reaktionszeit beträgt 5 - 10 Stunden, wobei eine 8-stündige Umsetzung in der Regel am günstigsten ist. Es hat sich als zweckmäßig erwiesen, dem Reaktionsgemisch gemahlenes Natriumchlorid und geringe Mengen Dimethylformamid zuzusetzen.

Da Phosphorpentachlorid bekanntlich aus Phosphortrichlorid und Chlor entsteht, ist es verständlich, daß anstelle von Phosphorpentachlorid auch ein Gemisch aus Chlor und Phosphortrichlorid im Molverhältnis 1 : 1 als Chlorierungsmittel angewandt werden kann.

Die Durchführung des erfindungsgemäßen Verfahrens erfordert keinen größeren technischen Aufwand. Erforderlich ist jedoch ein Autoklav, dessen Innenwand gegenüber dem Reaktionsgemisch beständig ist. Geeignet ist ein Autoklav, der beispielsweise aus einer Chrom-Nickel-Legierung ("Hastelloy") besteht.

Entgegen der in der weiter oben genannten Literaturstelle (Heterocyclic Compounds Vol. 5 l. c.) aufgestellten Behauptung, daß eine Chlorierung im Benzolkern die Überführung von Benzoxazolonen mit Phosphorpentachlorid in 2-Chlorbenzoxazole unmöglich mache, erwies eine gaschromatographisch-massenspektrometrische Untersuchung der Reaktionsgemische des von uns gefundenen Verfahrens, daß nur ca. 3 % Trichlorbenzoxazole und 4 - 5 % Kondensationsprodukte der Masse 320 neben einigen Komponenten mit bis zu 4 Chloratomen, jedoch in Mengen von jeweils nur ca. 1 %, und neben Komponenten mit höheren Massen (bis ca. 700) mit bis zu 6 Chloratomen auftreten.

Bei Einsatz von am Benzolring unsubstituiertem Benzoxalon erhält man neben 2-Chlorbenzoxazol bis zu gleich große Mengen 2,6-Dichlorbenzoxazol. Daraus läßt sich ableiten, daß die kernchlorierende Wirkung des Gemischs aus Phosphorpentachlorid und Phosphoroxidchlorid zunächst hauptsächlich auf die 6-Stellung gerichtet ist. Ist diese, wie beim Einsatz von 6-Chlorbenzoxazolon, bereits besetzt, so ist die kernchlorierende Wirkung des Gemisches aus Phosphorpentachlorid und Phosphoroxidchlorid nicht mehr von nachteiliger Bedeutung. Man erhält dann vielmehr ein definiertes, im Kern monochloriertes Produkt (2,6-Dichlorbenzoxazol). Zumindest für die Herstellung von 2,6-Dichlorbenzoxazol ist das in der genannten Literaturstelle Heterocyclic Compounds Vol. 5 genannte Argument nicht mehr sinnvoll und nur dazu angetan, ein Vorurteil gegen diese Reaktionsmöglichkeit aufzubauen.

Das als Ausgangsverbindung dienende 6-Chlorbenzoxazolon läßt sich auf einfache Weise durch Chlorieren von Benzoxazolon in verdünnter Salzsäure mit Hypochlorit bzw. in konz. Salzsäure mit Wasserstoffperoxid (also in Abwesenheit organischer Lösemittel) herstellen. (Die Ausbeute des 6-Chlorbenzoxazolons beträgt hierbei 82 % der Theorie, bezogen auf Benzoxazolon, und der Reingehalt 90 %).

Nachstehend seien Einzelheiten bzw. bevorzugte Ausführungsformen des erfindungsgemäßen Verfahrens beschrieben:

Da der Umgang mit Phosphorpentachlorid im Betrieb aus toxikologischen und sicherheitstechnischen Gründen bedenklich ist, kann man das Phosphorpentachlorid auch herstellen durch Eingasen von Chlor in Phosphoroxidchlorid, dem eine zum Chlor äquivalente Menge Phosphortrichlorid beigemischt ist. Beim erfindungemäßen Verfahren wird 1 Mol Chlorwasserstoff freigesetzt. Dadurch und durch den Dampfdruck des Phosphoroxidchlorids stellen sich bei der vorzugsweisen Reaktionstemperatur von 155 - 165°C Drucke von 6 - 12 bar ein.

Eine Milderung der Chlorierungsbedingungen durch Zusatz von Pyridin oder durch Zusatz von Wasser (d. h. Verwendung von Pyrophosphorylchlorid) verschlechtern das Ergebnis erheblich, weshalb davon abzusehen ist.

Bei der Aufarbeitung des Reaktionsgemisches erfolgt zunächst die destillative Regeneration des Phosphoroxidchlorids. Der Destillationsrückstand wird in Toluol-Wasser-Gemischen aufgenommen und von einem Bodenkörper abfiltriert, dessen Menge umso niedriger ist, je höher das Einsatzverhältnis von Phosphorpentachlorid/Phosphoroxidchlorid ist. Er enthält höher chlorierte phosphorsäurehaltige Verbindungen.

Nach Phasentrennung des Filtrats wird die Toluolphase mit wäßriger Bicarbonatlösung gewaschen, dann über Calciumchlorid getrocknet und von Calciumchlorid abfiltriert. Das Filtrat wird zur Regeneration des Toluols auf Rückstand destilliert. Der Destillationsrückstand enthält ca. 75 Gewichtsprozent 2,6-Dichlorbenzoxazol, entsprechend einer Rohausbeute von 60 % der Theorie (Gaschromatographie). Der Rest ist bis zu ca. 10 Gewichstprozent Toluol und ca. 5 % Kondensationsprodukt aus 6-Chlorbenzoxazolon und 2,6-Dichlorbenzoxazol der Masse 320. Die letztgenannte Nebenkomponente nimmt zu und der Chlorierungsgrad des Benzolrings nimmt ab mit Abnahme des Mengenverhältnisses von Phosphorpentachlorid zu Phosphoroxidchlorid.

Der Destillationsrückstand wird im Vakuum fraktioniert destilliert, wobei man 2,6-Dichlorbenzoxazol mit einem Reingehalt von mindestens 97 % (Fp. 40 - 44°C) in einer Ausbeute von über 90 % der Theorie, bezogen auf Rohprodukt, entsprechend 55 % der Theorie, bezogen auf 6-Chlorbenzoxazolon, erhält.

2,6-Dichlorbenzoxazol ist ein wertvolles Zwischenprodukt für die Herstellung von Pflanzenschutzwirkstoffen (DE-OS 2 640 730, 2 758 002 und 2 830 066; EP 0 062 905).

Beispiel 1

In einem Autoklav aus einer Nickel-Chrom-Legierung werden 600 Teile Phosphoroxidchlorid vorgelegt. Dann werden 35 Teile gemahlenes Natriumchlorid, 35 Teile umkristallisiertes 6-Chlorbenzoxazolon (Fp. 190 - 194°C), 1 Teil Dimethylformamid und 100 Teile Phosphorpentachlorid eingetragen. Man verschließt den Autoklav, heizt auf 155°C und rührt 8 Stunden bei 155°C nach. Es stellt sich ein Druck von ca. 8 bar ein. Darauf entspannt man den Autoklav bei 25°C und destilliert anschließend 640 Teile Phosphoroxidchlorid bis 120°C und 133 mbar ab.

Der Destillationsrückstand wird mit 200 Teilen Toluol und 200 Teilen Eiswasser verrührt und das Gemisch vom Bodenkörper (15 Teile) abfiltriert. Das Toluolfiltrat wird nach Abtrennen von der wäßrigen Phase mit 100 Teilen 5 %iger Natriumhydrogencarbonatlösung gewaschen. Die Toluol-Lösung wird nach Abtrennen der wäßrigen Phase über Calciumchlorid getrocknet. Das Toluol wird bei Normaldruck abdestilliert. Im Destillationsrückstand (28 Teile) werden durch kombinierte gaschromatographische und massenspektroskopische Messung 21 Teile 2,6-Dichlorbenzoxazol (Reingehalt 75 %), 2 Teile Toluol, 0,8 Teile Trichlorbenzoxazol (Masse 221), 0,1 Teile Tetrachlorbenzoxazol (Masse 255), 1,1 Teile Kondensationsprodukt $C_{14}H_6O_3N_2Cl_2$ (Masse: 320), 0,3 Teile Kondensationsprodukt $C_{14}H_5O_3N_2Cl_3$ (Masse: 354) gefunden. Der Rest besteht aus schwer bzw. nicht flüchtigen Anteilen. Der Gehalt an verseifbarem Chlor beträgt 16,5 % (theor. 18,9 %), der Gehalt an gesamtem Chlor 35,5 % (theor. 37,7 %).

Das Rohprodukt erstarrt bei 25°C zu einer festen kristallinen Masse. Die fraktionierte Destillation bei 3 mbar über eine auf 70°C beheizte Destillationsbrücke ergibt 19,4 Teile 97 %iges 2,6-Dichlorbenzoxazol, entsprechend 18,6 Teilen gerechnet 100 %= 50 % der Theorie, bezogen auf 6-Chlorbenzoxazolon (Fp 40 - 44°C).

Beispiel 2

Man verfährt, wie in Beispiel 1 beschrieben, jedoch unter Anwendung von 150 Teilen Phosphorpentachlorid und einer Reaktionszeit von nur 5 Stunden. Man erhält bei der destillativen Regeneration 620 Teile Phosphoroxidchlorid. Die Aufarbeitung der 90 Teile Destillationsrückstand ergibt

a) 12 Teile Filterrückstand (aus der Aufnahme in Toluol/Wasser);

b) 26 Teile rohes 2,6-Dichlorbenzoxazol (nach Abdestillieren des Toluols) mit einem Gehalt von 21 Teilen 2,6-Dichlorbenzoxazol.

**0153656**

der Gehalt an verseifbarem Chlor beträgt 19,5 %. Das Rohprodukt erstarrt bei 25°C zu einer festen kristallinen Masse.

Beispiel 3

Man setzt 35 Teile 6-Chlorbenzoxazolon mit 250 Teilen Phosphorpentachlorid und 450 Teilen Phosphoroxidchlorid 8 Stunden bei 165°C um. Der Druck beträgt schließlich 12 bar. Das Reaktionsgemisch wird langsam auf 2000 Teile Eis und 870 Teile Toluol, die intensiv gerührt werden, gegeben. Dabei bildet sich kein Bodenkörper. Die Toluolphase wird abgetrennt, mit Bicarbonatlösung und Wasser gewaschen, dann über Calciumchlorid getrocknet und filtriert. Nach Abdestillation des Toluols werden 34 Teile Destillationsrückstand erhalten. Er enthält 23 Teile 2,6-Dichlorbenzoxazol (entsprechend einem Reingehalt von 67 %), ca. 3 Teile Trichlorbenzoxazol (Masse 221), ca. 0,5 Teile Tetrachlorbenzoxazol (Masse 255), ca. 0,3 Teile Pentachlorbenzoxazol (Masse 289) und ca. 0,3 Teile Kondensationsprodukt $C_{14}H_6O_3N_2Cl_2$ der Masse 320. Der Rest enthält die schwer- bis nicht flüchtigen Anteile (darunter Komponenten mit Masse 700 und 6 Chloratomen).

Der Gehalt an verseifbarem Chlor beträgt 17,0 % (theor. 18,9 %), der Gehalt an gesamtem Chlor beträgt 41,7 % (theor. 37,7 %).

Das Rohprodukt erstarrt bei 25°C zu einer festen kristallinen Masse. Nach der fraktionierten Destillation werden 20,7 Teile 2,6-Dichlorbenzoxazol 100 % gerechnet= 55 % der Theorie, bezogen auf 6-Chlorbenzoxazolon, erhalten.

Beispiel 4

Man verfährt, wie in Beispiel 1 beschrieben, jedoch ohne Verwendung von Phosphorpentachlorid. Der Druck beträgt bei 160°C 6 bar, der Filterrückstand aus der Toluol/Wasser-Verrührung beträgt 11 Teile. Der Destillationsrückstand nach Abdestillation des Toluols beträgt nur 8 - 9 g und ist ein pulverförmiger Feststoff. Durch kombinierte gaschromatographische und massenspektrometrische Untersuchung findet man im Feststoff 75 % Kondensationsprodukt der Massen 320 und 354. Der Rest ist 6-Chlorbenzoxazolon (= Ausgangsprodukt). 2,6-Dichlorbenzoxazol konnte nicht nachgewiesen werden. Der Gehalt an verseifbarem Chlor beträgt nur 2,1 % bei einem Gesamtchlorgehalt von 20,2 %.

Beispiel 5

Man verfährt, wie in Beispiel 1 beschrieben, jedoch unter Verwendung von 35 Teilen eines nur 88 %igen 6-Chlorbenzoxazolons vom Fp. 178 - 181°C entsprechend 0,18 Mol (Rohprodukt aus der Chlorierung von Benzoxazolon). Die Aufarbeitung erfolgt wie in Beispiel 3 beschrieben. Es fällt kein unlöslicher Bodenkörper an. Man erhält 31 Teile Rohprodukt (nach Abdestillation des Toluols) mit 22 Teilen 2,6-Dichlorbenzoxazol (entsprechend einem Reingehalt von 70 %) und 8 Teilen spät eluierbaren Komponenten. Der Gehalt an verseifbarem Chlor beträgt 19,9 %.

Die Ausbeute beträgt 22 Teile, gerechnet 100 %= 0,116 Mol= 64 % der Theorie, bezogen auf 6-Chlorbenzoxazolon bzw. 52 % der Theorie, bezogen auf Benzoxazolon.

HOE 84/F 037
**0153656**

Patentanspruch:

Verfahren zur Herstellung von 2,6-Dichlorbenzoxazol, dadurch gekennzeichnet, daß man 6-Chlorbenzoxazolon in Gemischen aus Chlor, Phosphortrichlorid und Phosphoroxidchlorid bzw. in Gemischen aus Phosphorpentachlorid und Phosphoroxidchlorid bei Temperaturen von 150 bis 170°C unter Druck umsetzt, wobei das Gewichtsverhältnis von Chlor und Phosphortrichlorid zu Phosphoroxidchlorid bzw. von Phosphorpentachlorid zu Phosphoroxidchlorid 0,15 : 1 bis 0,60 : 1 beträgt.

**Europäisches Patentamt**

**EUROPÄISCHER RECHERCHENBERICHT**

| | EINSCHLÄGIGE DOKUMENTE | | EP 85101519.8 |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| D,A | EP - A1 - 0 043 573 (HOECHST)<br><br>* Zusammenfassung *<br><br>-- | 1 | C 07 D 263/58 |
| A | CHEMICAL ABSTRACTS, Band 63, Nr. 10, 8. November 1965, Columbus, Ohio, USA<br><br>M. FOA, A. RICCI, P.E. TODESCO, P. VIVARELLI "Reactivity of groups in the 2-position of benzoxazoles"<br>Spalte 13 236, Zusammenfassung-Nr. 13 236a<br><br>& Boll. Sci. Fac. Chim. Ind. Bologna 23(2-3), 89-98 (1965)<br><br>---- | | |
| | | | **RECHERCHIERTE SACHGEBIETE (Int. Cl.4)**<br><br>C 07 D 263/00 |

Der vorliegende Recherchenbericht wurde für alle Patentansprüche erstellt.

| Recherchenort | Abschlußdatum der Recherche | Prüfer |
|---|---|---|
| WIEN | 06-05-1985 | HAMMER |